# EUROPEAN PATENT APPLICATION

(11) **EP 1 698 340 A1**
(43) Date of publication of application: **06.09.2006**
(21) Application number: 04819406.2
(22) Date of filing: 25.11.2004
(51) Int. Cl.: A61K 31/675, A61P 7/10, A61P 9/00, A61P 9/04, A61P 9/10, A61P 13/12, A61P 25/04, A61P 25/08, A61P 29/00, A61P 35/00, A61P 43/00

(54) **T-TYPE CALCIUM CHANNEL INHIBITOR**

(30) Priority: 25.11.2003 JP 2003393880; 25.11.2003 JP 2003393884; 25.11.2003 JP 2003393886
(71) Applicant: Nissan Chemical Industries, Ltd., Chiyoda-ku, Tokyo 101-0054 (JP)
(72) Inventor: FURUKAWA, Taiji 11-7-628, Nishihori 4-chome, Saitama 3380832 (JP); YAMADA, Osamu c/o Nissan Chemical Industries,, Tsuboi-cho Funabashi-shi, Chiba 2748507 (JP); MATSUMOTO, Hiroo c/o Nissan Chemical Industries,, Tsuboi-cho Funabashi-shi, Chiba 2748507 (JP); YAMASHITA, Toru c/o Nissan Chemical Industries,, Tokyo 1010054 (JP); MASUDA, Yukinori c/o Nissan Chem. Industries, Ltd., Chiyoda-ku, Tokyo 101 0054 (JP)
(74) Representative: HOFFMANN EITLE
(86) International application number: PCT/JP2004/017490
(87) International publication number: WO 2005/051402

(57) **Abstract**

There is provided a T-type calcium channel blocker that is a compound of formula (1), a pharmaceutically acceptable salt thereof or a solvate thereof: 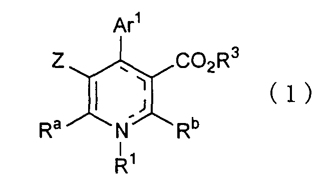 wherein
Ar¹ is phenyl group, pyridyl group, furyl group or 2,1,3-benzoxadiazol-4-yl group;
nitrogen-containing hetero ring moiety is 1,4-dihydropyridine ring or pyridine ring;
Z is a group of formula (2) 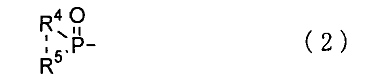 or CO₂R²;
R^{a} and R^{b} are independently of each other C₁₋₆alkyl group, ANR⁸R⁹, CH₂OANR⁸R⁹, or the like;
in case where the nitrogen-containing hetero ring moiety is 1,4-dihydropyridine ring,
R¹ is C₁₋₆alkyl group, ANR⁸R⁹, AN(CH₂CH₂)₂NR⁸, AN(CH₂CH₂)₂O, AOR⁸ or benzyl group;
R³ is hydrogen atom, C₁₋₂₀alkyl group, ANR⁸R⁹, a group of formula 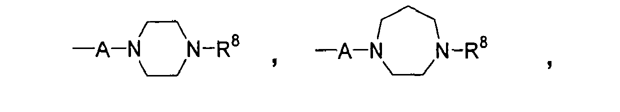 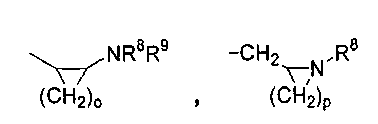 or 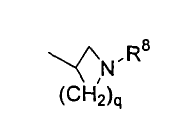 or the like.

## Description

### Technical Field

The present invention relates to a pyridine compound and 1,4-dihydropyridine compound having a substituent at 1-position that show a selective T-type calcium chanel blocking action, and to therapeutic or preventive agents against diseases for which T-type calcium channel blocking action is effective.

### Background Art

It is known that a number of 1,4-dihydropyridine compounds show oral antihypertensive action and are effective for improvement of cardiovascular diseases such as angia pectoris, cerebrovascular disease, hypertension or the like (see, for example Patent Documents 1-12). The above-mentioned effect that these compounds show is owing to vasodilation mainly based on L-type calcium channel blocking action.

Recently, compounds having T-type calcium channel blocking action in addition to L-type calcium channel blocking action were found from among 1,4-dihydropyridine compounds (see, for example, Non-patent Documents 1 and 2).

It is reported that the activation of T-type calcium channel participates in occurrence of hypercardia (see, for example Non-patent Document 3), heart failure (see, for example Non-patent Document 3), cardiomyopathy (see, for example Non-patent Document 4), tachyarrhythmia represented by atrial fibrillation (see, for example Non-patent Document 5), arterial sclerosis (see, for example Non-patent Document 6), renal disorder represented by nephritis/nephropathy (see, for example Non-patent Document 7), renal insufficiency (see, for example Non-patent Document 7), inflammation and edema (see, for example Non-patent Document 8), hyper-aldosteronism (see, for example Non-patent Document 9), neurogenic pain (see, for example Non-patent Document 10), epilepsy (see, for example Non-patent Document 11) and cancer (see, for example Non-patent Document 12). Therefore, it is thought that T-type calcium channel blockers are effective for therapy or prevention of these diseases.

On the other hand, it is reported that 1,4-dihydropyridine-5-phosphonate derivatives and pyridine-5-phosphonate derivatives have an augmentation action of cardioprotective effectve against ischemia-reperfusion (see, for example Patent Document 14). However, the possibility of these compounds as T-type calcium channel blockers is not mentioned therein.
Patent Document 1: JP 61-30591 A (1986)
Patent Document 2: JP 60-69089 A (1985)
Patent Document 3: JP 1-275591 A (1989)
Patent Document 4: JP 61-63688 A (1986)
Patent Document 5: JP 63-233992 A (1988)
Patent Document 6: JP 62-169795 A (1987)
Patent Document 7: JP 62-169796 A (1987)
Patent Document 8: JP 58-167569 A (1983)
Patent Document 9: JP 55-301 A (1980)
Patent Document 10: JP 62-174017 A (1987)
Patent Document 11: JP 60-97956 A (1985)
Patent Document 12: JP 49-108082 A (1974)
Patent Document 13: JP 2-138221 A (1990)
Patent Document 14: JP 2002-226376 A (2002)
Non-patent Document 1: Mol. Pharmacol., 61, p.649-658, (2002)
Non-patent Document 2: Masumiya H. et al.: Eur. J. Pharmacol. 335, p.15-21 (1997)
Non-patent Document 3: Mulder P. et al.: J. Am. Coll. Cardiol. 29, p.416-421 (1997)
Non-patent Document 4: Villame J. et al.: Cardiovasc Drugs Ther. 15, p.41-48 (2001)
Non-patent Document 5: Fareh S. et al.: Circulation 100, p.2191-2197 (1999)
Non-patent Document 6: Noll G. and Luscher T. F.: Cardiology 89, p.10-15 (1998)
Non-patent Document 7: Baylis C. et al.: Am. J. Kidney Dis. 38, p.1292-1297 (2001)
Non-patent Document 8: Bilici D. et al.: Pharmacol. Res. 44, p.527-531 (2001)
Non-patent Document 9: Lenglet S. et al.: Endocrinology 143, p.1748-60 (2002)
Non-patent Document 10: McCallum J. B. et al.: Anesthesiology 98, p.209-216 (2003)
Non-patent Document 11: Porcello D. M. et al.: J. Neurophysiol. 89, p.177-185 (2003)
Non-patent Document 12: Mc Calmont W. F. et al.: Bioorg. Med. Chem. Lett. 14(14), p.3691-3695 (2004)

### Disclosure of Invention

### Problem to be solved by the Invention

In the therapy of the above-mentioned diseases based on T-type calcium blocking action, the followings are feared: 1,4-dihydropyridine compounds have a possibility that the influence thereby on a strong vasodilation and cardiac function based on L-type calcium channel blocking action simultaneously caused becomes inhibition factors in the therapy; and these compounds have a possibility to cause lowering in Quality of Life, such as occurrence of headache, flash, dizziness, edema or the like based on vasodilation. Therefore, it is considered very useful to find T-type calcium channel blockers having a weak or little L-type calcium channel blocking action, as therapeutic agents of the above-mentioned diseases.
Means for solving the Problem

The present inventors eagerly investigated in order to solve the above-mentioned problems. As a result of it, they found that the oxidation of 1,4-dihydropyridine compounds to pyridine or the introduction of substituent at 1-position of 1,4-dihydropyridine compounds leads to the production of compounds in which L-type calcium channel blocking action is weakened and T-type calcium channel blocking action is maintained to some extent, and consequently a selective blocking action against T-type calcium channel is maintained at the approximately same level. Consequently, they completed the present invention.

That is, the present invention provides the followings:
1. A T-type calcium channel blocker that is a compound of formula (1), a pharmaceutically acceptable salt thereof or a solvate thereof: 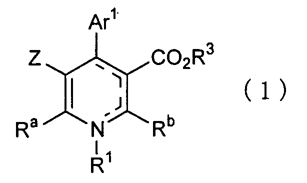 wherein
   Ar¹ is phenyl group, pyridyl group, furyl group or 2,1,3-benzoxadiazol-4-yl group (the phenyl group, pyridyl group, furyl group and 2,1,3-benzoxadiazol-4-yl group may be arbitrarily substituted with one or two substituents selected from NO₂, CF₃, Br, Cl, F, C₁₋₂₀alkyl group, OH, OR⁶, OCHF₂, COOR⁶, NH₂, NHR⁶, NR⁶R⁷, CONH₂, CONHR⁶, CONR⁶R⁷, COSR⁶, SR⁶, S(O)R⁶, S(O)₂R⁶, SO₃H, SO₃R⁶, SO₂NH₂, SO₂NHR⁶, SO₂NR⁶R⁷, CN and phenyloxy group, wherein R⁶ and R⁷ are independently of each other C₁₋₆alkyl group;
   nitrogen-containing hetero ring moiety is 1,4-dihydropyridine ring or pyridine ring;
   Z is a group of formula (2) 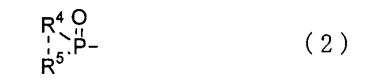 wherein R⁴ and R⁵ are independently of each other OH, C₁₋₆alkoxy group,
   C₃₋₆alkenyloxy group, C₃₋₆alkynyloxy group, OAr², OANR⁶R⁷, OAN(CH₂Ar²)R⁶, OAOR⁶, OACN, NH₂, NHR⁶, NR⁶R⁷, 1-pyperidinyl group or 1-pyrrolidinyl group, or
   R⁴ and R⁵ together are OYO, NHYO, R⁶NYO, NHYNH, R⁶NYNH or R⁶NYNR⁷ wherein R⁶ and R⁷ are as defined above,
   Ar² is phenyl group (the phenyl group may be arbitrarily substituted with halogen atom, C₁₋₃alkyl group or C₁₋₃alkoxy group),
   A is C₂₋₆alkylene group (the C₂₋₆alkylene group may be arbitrarily substituted with
   C₁₋₃alkyl group or Ar²), and
   Y is straight-chain C₂₋₄alkylene group (the C₂₋₄alkylene group may be arbitrarily substituted with C₁₋₆alkyl group, C₁₋₆alkoxy group, C₁₋₆alkoxycarbonyl group or Ar²), or Z is CO₂R², wherein R² is C₁₋₆alkyl group (the C₁₋₆alkyl group may be arbitrarily substituted with C₁₋₃alkoxy group);
   R^{a} and R^{b} are independently of each other C₁₋₆alkyl group, ANR⁸R⁹, CH₂OANR⁸R⁹, Ar², CH=CHAr², CH₂CH(OH)Ar², CHO, CN, CH₂OH, CH₂OR⁸, AN(CH₂CH₂)₂NR⁸ or NR⁸R⁹, wherein R⁸ and R⁹ are independently of each other hydrogen atom, C₁₋₆alkyl group (the C₁₋₆alkyl group may be arbitrarily substituted with phenyl group, wherein the phenyl group may be arbitrarily substituted with C₁₋₆alkoxy group or halogen atom) or phenyl group (the phenyl group may be arbitrarily substituted with C₁₋₆alkoxy group or halogen atom),
   Ar² and A are as defined above;
   in case where the nitrogen-containing hetero ring moiety is 1,4-dihydropyridine ring, R¹ is C₁₋₆alkyl group, ANR⁸R⁹, AN(CH₂CH₂)₂NR⁸, AN(CH₂CH₂)₂O, AOR⁸ or benzyl group, wherein R⁸, R⁹ and A are as defined above; and
   R³ is hydrogen atom, C₁₋₂₀alkyl group, C₂₋₆alkenyl group or C₂₋₆alkynyl group (C₁₋₂₀alkyl group, C₂₋₆alkenyl group and C₂₋₆alkynyl group may be arbitrarily substituted with phenyl group, wherein the phenyl group may be arbitrarily substituted with C₁₋₆alkoxy group or halogen atom), ANR⁸R⁹ or a group of formula 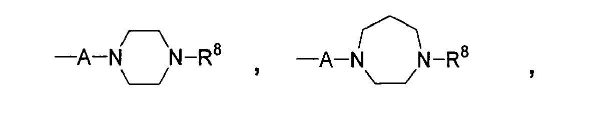 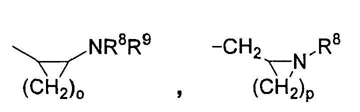 or 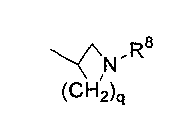 wherein R⁸, R⁹ and A are as defined above,
   o and p are independently of each other 3 or 4, and
   q is 1,2 or 3;
2. The T-type calcium channel blocker as set forth in 1., wherein R³ is ANR⁸R⁹ or a group of formula 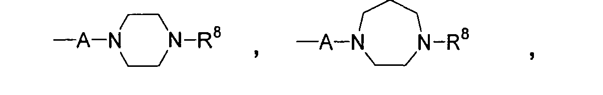 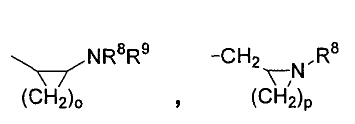 or 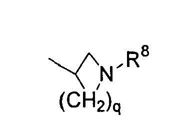 wherein R⁸, R⁹, A, o, q and p are as defined above; and
   R⁵ is C₁₋₆alkyl group;
3. The T-type calcium channel blocker as set forth in 2., wherein R^{b} is C₁₋₆alkyl group, CN or NH₂;
4. The T-type calcium channel blocker as set forth in 1., wherein R^{b} is ANR⁸R⁹, CH₂OANR⁸R⁹ or CH₂CH₂N(CH₂CH₂)₂NR⁸, wherein
   A, R⁸ and R⁹ are as defined above;
   R³ is C₁₋₂₀alkyl group, C₂₋₆alkenyl group or C₂₋₆alkynyl group (C₁₋₂₀alkyl group,
   C₂₋₆alkenyl group and C₂₋₆alkynyl group may be arbitrarily substituted with phenyl group, wherein the phenyl group may be arbitrarily substituted with C₁₋₆alkoxy group or halogen atom); and
   R⁵ is C₁₋₆alkyl group;
5. The T-type calcium channel blocker as set forth in any one of 1. to 4., wherein the nitrogen-containing hetero ring moiety is 1,4-dihydropyridine ring; and
   Z is a group of formula (2);
6. The T-type calcium channel blocker as set forth in 5., wherein R⁴ and R⁵ together are OYO, NHYO, R⁶NYO, NHYNH, R⁶NYNH or R⁶NYNR⁷, wherein
   Y is straight-chain C₂₋₄alkylene group (the C₂₋₄alkylene group may be substituted with C₁₋₆alkyl group, C₁₋₆alkoxy group, C₁₋₆alkoxycarbonyl group or Ar²);
7. The T-type calcium channel blocker as set forth in 6., wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group;
8. The T-type calcium channel blocker as set forth in any one of 1. to 4., wherein the nitrogen-containing hetero ring moiety is pyridine ring; and
   Z is a group of formula (2);
9. The T-type calcium channel blocker as set forth in 8., wherein R⁴ and R⁵ together are OYO, NHYO, R⁶NYO, NHYNH, R⁶NYNH or R⁶NYNR⁷, wherein
   Y is straight-chain C₂₋₄alkylene group (the C₂₋₄alkylene group may be arbitrarily substituted with C₁₋₆alkyl group, C₁₋₆alkoxy group, C₁₋₆alkoxycarbonyl group or Ar²);
10. The T-type calcium channel blocker as set forth in 9., wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group;
11. The T-type calcium channel blocker as set forth in any one of 1. to 4., wherein the nitrogen-containing hetero ring moiety is 1,4-dihydropyridine ring; and
   Z is CO₂R²;
12. The T-type calcium channel blocker as set forth in 11., wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group;
13. The T-type calcium channel blocker as set forth in any one of 1. to 4., wherein the nitrogen-containing hetero ring moiety is pyridine ring; and
   Z is CO₂R²;
14. The T-type calcium channel blocker as set forth in 13., wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group;
15. A pharmaceutical containing the T-type calcium channel blocker as set forth in 1.;
16. The pharmaceutical as set forth in 15., wherein the pharmaceutical is a therapeutic or preventive agent against a disease for which T-type calcium channel blocking action is effective;
17. The pharmaceutical as set forth in 16., wherein the disease is hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer;
18. A method for preventing or treating hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer, comprising administering an effective amount of the compound of formula (1), a pharmaceutically acceptable salt thereof or a solvate thereof as set forth in 1.; and
19. Use of the compound of formula (1), a pharmaceutically acceptable salt thereof or a solvate thereof as set forth in 1. for the manufacture of a preventive agent or a therapeutic agent for hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer.

### Best Mode for Carrying Out the Invention

Hereinafter, the present invention is described in further detail.
In the meantime, "n" means normal, "i" means iso, "s" means secondary, "t" means tertiary, "c" means cyclo and "Ph" means phenyl group in this specification.

Each substituent stated in this specification is described.

Halogen atom includes fluorine atom, chlorine atom, bromine atom and iodine atom.

C₁₋₃ alkyl group includes straight-chain, branched or cyclic ones, for example methyl group, ethyl group, n-propyl group, i-propyl group and c-propyl group, etc.

C₁₋₆alkyl group includes straight-chain, branched or cyclic ones, for example in addition to the above-mentioned groups for C₁₋₃ alkyl group, n-butyl group, i-butyl group, s-butyl group, t-butyl group, c-butyl group, n-pentyl group, 1-methyl-n-butyl group, 2-methyl-n-butyl group, 3-methyl-n-butyl group, 1,1-dimethyl-n-propyl group, c-pentyl group, 2-methyl-c-butyl group, n-hexyl group, 1-methyl-n-pentyl group, 2-methyl-n-pentyl group, 1,1-dimethyl-n-butyl group, 1-ethyl-n-butyl group, 1,1,2-trimethyl-n-propyl group, c-hexyl group, 1-methyl-c-pentyl group, 1-ethyl-c-butyl group and 1,2-dimethyl-c-butyl group, etc.

C₁₋₂₀ alkyl group includes straight-chain, branched or cyclic ones, for example in addition to the above-mentioned groups for C₁₋₆alkyl group, n-heptyl group, 2-c-pentylethyl group, n-octyl group, 2-c-hexylethyl group, 3-c-pentyl-n-propyl group, n-nonyl group, 3-c-hexyl-n-propyl group, 4-c-pentyl-n-butyl group, n-decyl group, 4-c-hexyl-n-butyl group, 5-c-pentyl-n-pentyl group, n-undecyl group, 5-c-hexyl-n-pentyl group, 6-c-pentyl-n-hexyl group, n-dodecyl group, n-tridecyl group, n-tetradecyl group, n-pentadecyl group, n-hexadecyl group, n-heptadecyl group, n-octadecyl group, n-nonadecyl group and n-eicosyl, etc.

C₂₋₆alkenyl group includes straight-chain or branched ones, for example ethenyl group, 1-propenyl group, 2-propenyl group, 1-methyl-1-ethenyl group, 1-butenyl group, 2-butenyl group, 3-butenyl group, 2-methyl-1-propenyl group, 2-methyl-2-propenyl group, 1-ethylethenyl group, 1-methyl-1-propenyl group, 1-methyl-2-propenyl group, 1-pentenyl group, 2-pentenyl group, 3-pentenyl group, 4-pentenyl group, 1-n-propylethenyl group, 1-methyl-1-butenyl group, 1-methyl-2-butenyl group, 1-methyl-3-butenyl group, 2-ethyl-2-propenyl group, 2-methyl-1-butenyl group, 2-methyl-2-butenyl group, 2-methyl-3-butenyl group, 3-methyl-1-butenyl group, 3-methyl-2-butenyl group, 3-methyl-3-butenyl group, 1,1-dimethyl-2-propenyl group, 1-i-propylethenyl group, 1,2-dimethyl-1-propenyl group, 1,2-dimethyl-2-propenyl group, 1-hexenyl group, 2-hexenyl group, 3-hexenyl group, 4-hexenyl group, 5-hexenyl group, 1-methyl-1-pentenyl group, 1-methyl-2-pentenyl group, 1-methyl-3-pentenyl group, 1-methyl-4-pentenyl group, 1-n-butylethenyl group, 2-methyl-1-pentenyl group, 2-methyl-2-pentenyl group, 2-methyl-3-pentenyl group, 2-methyl-4-pentenyl group, 2-n-propyl-2-propenyl group, 3-methyl-1-pentenyl group, 3-methyl-2-pentenyl group, 3-methyl-3-pentenyl group, 3-methyl-4-pentenyl group, 3-ethyl-3-butenyl group, 4-methyl-1-pentenyl group, 4-methyl-2-pentenyl group, 4-methyl-3-pentenyl group, 4-methyl-4-pentenyl group, 1,1-dimethyl-2-butenyl group, 1,1-dimethyl-3-butenyl group, 1,2-dimethyl-1-butenyl group, 1,2-dimethyl-2-butenyl group, 1,2-dimethyl-3-butenyl group, 1-methyl-2-ethyl-2-propenyl group, 1-s-butylethenyl group, 1,3-dimethyl-1-butenyl group, 1,3-dimethyl-2-butenyl group, 1,3-dimethyl-3-butenyl group, 1-i-butylethenyl group, 2,2-dimethyl-3-butenyl group, 2,3-dimethyl-1-butenyl group, 2,3-dimethyl-2-butenyl group, 2,3-dimethyl-3-butenyl group, 2-i-propyl-2-propenyl group, 3,3-dimethyl-1-butenyl group, 1-ethyl-1-butenyl group, 1-ethyl-2-butenyl group, 1-ethyl-3-butenyl group, 1-n-propyl-1-propenyl group, 1-n-propyl-2-propenyl group, 2-ethyl-1-butenyl group, 2-ethyl-2-butenyl group, 2-ethyl-3-butenyl group, 1,1,2-trimethyl-2-propenyl group, 1-t-butylethenyl group, 1-methyl-1-ethyl-2-propenyl group, 1-ethyl-2-methyl-1-propenyl group, 1-ethyl-2-methyl-2-propenyl group, 1-i-propyl-1-propenyl group and 1-i-propyl-2-propenyl group, etc.

C₂₋₆ alkynyl group includes straight-chain or branched ones, for example ethynyl group, 1-propynyl group, 2-propynyl group, 1-butynyl group, 2-butynyl group, 3-butynyl group, 1-methyl-2-propynyl group, 1-pentynyl group, 2-pentynyl group, 3-pentynyl group, 4-pentynyl group, 1-methyl-2-butynyl group, 1-methyl-3-butynyl group, 2-methyl-3-butynyl group, 3-methyl-1-butynyl group, 1,1-dimethyl-2-propynyl group, 2-ethyl-2-propynyl group, 1-hexynyl group, 2-hexynyl group, 3-hexynyl group, 4-hexynyl group, 5-hexynyl group, 1-methyl-2-pentynyl group, 1-methyl-3-pentynyl group, 1-methyl-4-pentynyl group, 2-methyl-3-pentynyl group, 2-methyl-4-pentynyl group, 3-methyl-1-pentynyl group, 3-methyl-4-pentynyl group, 4-methyl-1-pentynyl group, 4-methyl-2-pentynyl group, 1,1-dimethyl-2-butynyl group, 1,1-dimethyl-3-butynyl group, 1,2-dimethyl-3-butynyl group, 2,2-dimethyl-3-butynyl group, 3,3-dimethyl-1-butynyl group, 1-ethyl-2-butynyl group, 1-ethyl-3-butynyl group, 1-n-propyl-2-propynyl group, 2-ethyl-3-butynyl group, 1-methyl-1-ethyl-2-propynyl group and 1-i-propyl-2-propynyl group, etc.

C₁₋₃alkoxy group includes straight-chain, branched or cyclic ones, for example methoxy group, ethoxy group, n-propoxy group, i-propoxy group and c-propoxy group, etc.

C₁₋₆alkoxy group includes straight-chain, branched or cyclic ones, for example, in addition to the above-mentioned groups for C₁₋₃ alkoxy group, n-butoxy group, i-butoxy group, s-butoxy group, t-butoxy group, c-butoxy group, n-pentyloxy group, 1-methyl-n-butoxy group, 2-methyl-n-butoxy group, 3-methyl-n-butoxy group, 1,1-dimethyl-n-propoxy group, c-pentyloxy group, 2-methyl-c-butoxy group, n-hexyloxy group, 1-methyl-n-pentyloxy group, 2-methyl-n-pentyloxy group, 1,1-dimethyl-n-butoxy group, 1-ethyl-n-butoxy group, 1, 1, 2-trimethyl-n-propoxy group, c-hexyloxy group, 1-methyl-c-pentyloxy group, 1-ethyl-c-butoxy group and 1,2-dimethyl-c-butoxy group, etc.

C₃₋₆ alkenyloxy group includes straight-chain or branched ones, for example 2-propenyloxy group, 2-butenyloxy group, 3-butenyloxy group, 2-methyl-2-propenyloxy group, 1-methyl-2-propenyloxy group, 2-pentenyloxy group, 3-pentenyloxy group, 4-pentenyloxy group, 1-methyl-2-butenyloxy group, 1-methyl-3-butenyloxy group, 2-ethyl-2-propenyloxy group, 2-methyl-2-butenylooxy group, 2-methyl-3-butenyloxy goup, 3-methyl-2-butenyloxy group, 3-methyl-3-butenyloxy group, 1,1-dimethyl-2-propenyloxy group, 1,2-dimethyl-2-propenyloxy group, 2-hexenyloxy group, 3-hexenyloxy group, 4-hexenyloxy group, 5-hexenyloxy group, 1-methyl-2-pentenyloxy group, 1-methyl-3-pentenyloxy group, 1-methyl-4-pentenyloxy group, 2-methyl-2-pentenyloxy group, 2-methyl-3-pentenyloxy group, 2-methyl-4-pentenyloxy group, 2-n-propyl-2-propenyloxy group, 3-methyl-2-pentenyloxy group, 3-methyl-3-pentenyloxy group, 3-methyl-4-pentenyloxy group, 3-ethyl-3-butenyloxy group, 4-methyl-2-pentenyloxy group, 4-methyl-3-pentenyloxy group, 4-methyl-4-pentenyloxy group, 1,1-dimethyl-2-butenyloxy group, 1,1-dimethyl-3-butenyloxy group, 1,2-dimethyl-2-butenyloxy group, 1,2-dimethyl-3-butenyloxy group, 1-methyl-2-ethyl-2-propenyloxy group, 1,3-dimethyl-2-butenyloxy group, 1,3-dimethyl-3-butenyloxy group, 2,2-dimethyl-3-butenyloxy group, 2,3-dimethyl-2-butenyloxy group, 2,3-dimethyl-3-butenyloxy group, 2-1-propyl-2-propenyloxy group, 1-ethyl-2-butenyloxy group, 1-ethyl-3-butenyloxy group, 1-n-propyl-2-propenyloxy group, 2-ethyl-2-butenyloxy group, 2-ethyl-3-butenyloxy group, 1,1,2-trimethyl-2-propenyloxy group, 1-methyl-1-ethyl-2-propenyloxy group, 1-ethyl-2-methyl-2-propenyloxy group, 1-i-propyl-2-propenyloxy group, etc.

C₃₋₆ alkynyloxy group includes straight-chain or branched ones, for example 2-propynyloxy group, 2-butynyloxy group, 3-butynyloxy group, 1-methyl-2-propynyloxy group, 2-pentynyloxy group, 3-pentynyloxy group, 4-pentynyloxy group, 1-methyl-2-butynyloxy group, 1-methyl-3-butynyloxy group, 2-methyl-3-butynyloxy group, 1,1-dimethyl-2-propynyloxy group, 2-ethyl-2-propynyloxy group, 2-hexynyloxy group, 3-hexynyloxy group, 4-hexynyloxy group, 5-hexynyloxy group, 1-methyl-2-pentynyloxy group, 1-methyl-3-pentynyloxy group, 1-methyl-4-pentynyloxy group, 2-methyl-3-pentynyloxy group, 2-methyl-4-pentynyloxy group, 3-methyl-4-pentynyloxy group, 4-methyl-2-pentynyloxy group, 1,1-dimethyl-2-butynyloxy group, 1,1-dimethyl-3-butynyloxy group, 1,2-dimethyl-3-butynyloxy group, 2,2-dimethyl-3-butynyloxy group, 1-ethyl-2-butynyloxy group, 1-ethyl-3-butynyloxy group, 1-n-propyl-2-propynyloxy group, 2-ethyl-3-butynyloxy group, 1-methyl-1-ethyl-2-propynyloxy group, 1-i-propyl-2-propynyloxy group, etc.

C₁₋₆ alkoxycarbonyl group includes straight-chain, branched or cyclic ones, for example methoxycarbony group, ethoxycarbony group, n-propoxycarbony group, i-propoxycarbony group, c-propoxycarbonyl group, n-butoxycarbonyl group, i-butoxycarbonyl group, s-butoxycarbonyl group, t-butoxycarbonyl group, c-butoxycarbonyl group, n-pentyloxycarbonyl group, 1-methyl-n-butoxycarbonyl group, 2-methyl-n-butoxycarbonyl group, 3-methyl-n-butoxycarbonyl group, 1,1-dimethyl-n-propoxycarbonyl group, c-pentyloxycarbonyl group, 2-methyl-c-butoxycarbonyl group, n-hexyloxycarbonyl group, 1-methyl-n-pentyloxycarbonyl group, 2-methyl-n-pentyloxycarbonyl group, 1,1-dimethyl-n-butoxycarbonyl group, 1-ethyl-n-butoxycarbonyl group, 1,1,2-trimethyl-n-propoxycarbonyl group, c-hexyloxycarbonyl group, 1-methyl-c-pentyloxycarbonyl group, 1-ethyl-c-butoxycarbonyl group, 1,2-dimethyl-c-butoxycarbonyl group, etc.

C₂₋₄alkylene group includes ethylene group, propylene group, butylene group, etc. C₂₋₆alkylene group includes for example in addition to the above-mentioned groups for C₂₋₄alkylene group, pentylene group, hexylene group, etc.

Preferable R² include the following groups:
1. Methyl group, ethyl group, i-propyl group, i-butyl group and methoxyethyl group; and
2. Methyl group and ethyl group.

Preferable groups of formula (2) include the following groups:
1. Dimethylphosphonyl group, diethylphosphonyl group, 2-oxo-1,3,2-dioxaphosphorinan-2-yl group, 5,5-dimethyl-2-oxo-1,3,2-dioxaphosphorinan-2-yl group and 4,6-dimethyl-2-oxo-1,3,2-dioxaphosphorinan-2-yl group; and
2. Diethylphosphonyl group, 5,5-dimethyl-2-oxo-1,3,2-dioxaphosphorinan-2-yl group and 4,6-dimethyl-2-oxo-1,3,2-dioxaphosphorinan-2-yl group.

Preferable Ar¹ includes the following groups:
1. Phenyl group, 4-nitrophenyl group, 3-nitrophenyl group, 2-nitrophenyl group, 4-chlorophenyl group, 3-chlorophenyl group, 2-chlorophenyl group, 4-methoxyphenyl group, 3-methoxyphenyl group, 2-methoxyphenyl group, 4-trifluoromethylphenyl group, 3-trifluoromethylphenyl group, 2-trifluoromethylphenyl group, 4-pyridyl group, 3-pyridyl group, 2-pyridyl group and 2,3-dichlorophenyl group;
2. Phenyl group, 3-nitrophenyl group, 2-nitrophenyl group, 3-chlorophenyl group, 2-chlorophenyl group, 3-methoxyphenyl group, 2-methoxyphenyl group, 3-trifluoromethylphenyl group, 2-trifluoromethylphenyl group, 4-pyridyl group, 3-pyridyl group, 2-pyridyl group and 2,3-dichlorophenyl group;
3. Phenyl group, 3-nitrophenyl group, 2-nitrophenyl group, 3-chlorophenyl group, 2-chlorophenyl group, 3-methoxyphenyl group, 2-methoxyphenyl group, 3-trifluoromethylphenyl group, 2-trifluoromethylphenyl group, 4-pyridyl group, 3-pyridyl group and 2-pyridyl group; and
4. Phenyl group, 3-nitrophenyl group, 2-nitrophenyl group, 3-methoxyphenyl group, 2-methoxyphenyl group, 3-trifluoromethylphenyl group, 2-trifluoromethylphenyl group, 4-pyridyl group, 3-pyridyl group and 2-pyridyl group.

Preferable R^{a} includes the following groups:
1. C₁₋₆ alkyl group; and
2. Methyl group.

Preferable R^{b} includes the following groups:
1. C₁₋₆alkyl group, CN and NH₂;
2. Methyl group, CN and NH₂;
3. ANR⁸R⁹, CH₂OANR⁸R⁹ and CH₂CH₂N(CH₂CH₂)₂NR⁸;
4. CH₂OANR⁸R⁹; and
5. CH₂OCH₂CH₂NH₂.

Preferable R³ includes the following groups:
1. C₁₋₂₀alkyl group, C₂₋₆alkenyl group and C₂₋₆alkynyl group (the C₁₋₂₀alkyl group, C₂₋₆ alkenyl group and C₂₋₆ alkynyl group may be arbitrarily substituted with phenyl group, wherein the phenyl group may be arbitrary substituted with C₁₋₆alkoxy group or halogen atom);
2. Methyl group, ethyl group, i-propyl group, i-butyl group and methoxyethyl group;
3. ANR⁸R⁹ and groups of formulae 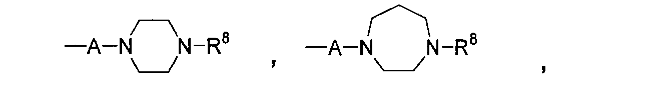 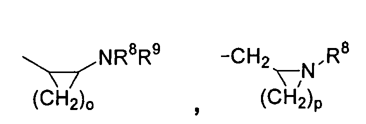 and 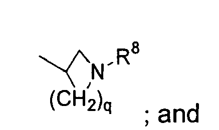
4. Groups of formulae 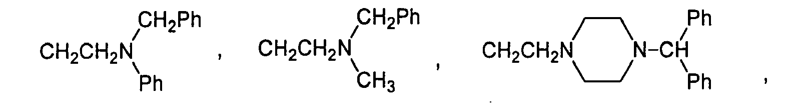 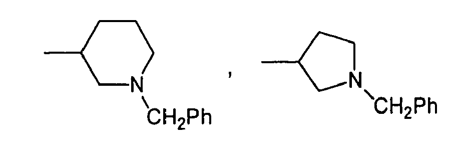 and

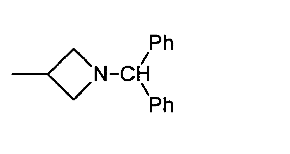

Preferable R¹ includes the following groups:
1. AN(CH₂CH₂)₂O; and
2. CH₂CH₂N(CH₂CH₂)₂O.

In case where the compound of formula (1) of the present invention is a compound that can form a salt, the pharmaceutically acceptable salt thereof can be also used as T-type calcium channel blocker. In case where the compound of formula (1) is a compound that can form a solvate, the solvate thereof can be also used as T-type calcium channel blocker.

The pharmaceutically acceptable salt includes hydrochlorides, hydrobromides, sulfates, methanesulfonates, acetates, benzoates, tartrates, phosphates, lactates, maleates, fumarates, malates, gluconates, salicylates and the like.

Preferably, hydrochlorides and methanesulfonates may be mentioned.

The solvates are not specifically limited so long as they are pharmaceutically acceptable, and concretely include hydrates and a solvate with ethanol, and the like.

T-type calcium channel blockers that are the compound of formula (1), a pharmaceutically acceptable salt thereof or a solvate thereof according to the present invention, pharmaceuticals containing the T-type calcium channel blockers, or therapeutic agents or preventive agents against diseases for which T-type calcium channel blocking action is effective, can be generally administered in oral administration forms such as tablets, capsules, powders, granules, pills, syrups and the like, permucosal absorption preparations such as intrarectal administration preparations, transnasal absorption preparations, transvaginal absorption preparations and the like, transpulmonary absorption preparations, inhalants, ophthalmic solutions, percutaneous absorption preparations or injections. The present preparations can be administered as a simple therapeutic agent or as a mixture with other therapeutic agent. The compound of formula (1) according to the present invention may be administered as a single item but are generally administered in a form of pharmaceutical composition. These preparations can be produced according to any conventional method by adding pharmacologically and pharmaceutically acceptable additives. That is, for oral preparations, additives such as excipients, lubricants, binders, disintegrators, humectants, plasticizers, coating agents and the like can be used. Oral liquids may be in a form of aqueous or oily suspension, solution, emulsion, syrup, elixir and the like, or be provided as a dry syrup that is prepared with water or other appropriate solvent prior to use. The above-mentioned liquids may contain conventional additives such as suspending agents, perfumes, diluents or emusifiers. When it is administered intrarectally, it can be administered as a suppository. The suppository may contain suitable base ingredients such as cocoa fats, lauric fats, macrogol, glycerogelatin, witepsol, sodium stearate or a mixture thereof, and optionally emulsifiers, suspending agents, preservatives and the like. For the injections, the followings are used: resolvents or solubilizing agents, such as distilled water for injections that can constitute aqueous dosage form or on use-dissolved type dosage form, saline, 5% glucose solution, propylene glycol and the like, pharmaceutical ingredients such as pH adjusters, isotonizing agents, stabilizers and the like.

When the pharmaceuticals of the present invention are administered to human, the dosage is determined depending on age or state of the patient. In case where the patient is adult, oral preparations or intrarectal administration is carried out in an amount of about 0.1 mg to 1000 mg per day per body, and an injection is administered in an amount of about 0.05 mg to 500 mg per day per body. These values are merely examples, and the dosage is determined according to the condition of a patient.

The scene which the present invention is applied includes the scene which the use of the compounds having T-type calcium channel blocking activity is expected to improve the condition of the disease. Concretely, the compounds of the present invention are effective for therapy or prevention of hypercardia, heart failure, cardiomyopathy, tachyarrhythmia represented by atrial fibrillation, arterial sclerosis, renal disorder represented by nephritis/nephropathy, renal insufficiency, inflammation and edema, hyper-aldosteronism, neurogenic pain, epilepsy, cancer and the like.

The compounds of formula (1) according to the present invention can be produced by oxidizing 1,4-dihydropyridine compounds or introducing a substituent at 1-position of 1,4-dihydropyridine compounds.

The production process thereof is shown in Scheme 1.

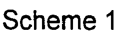

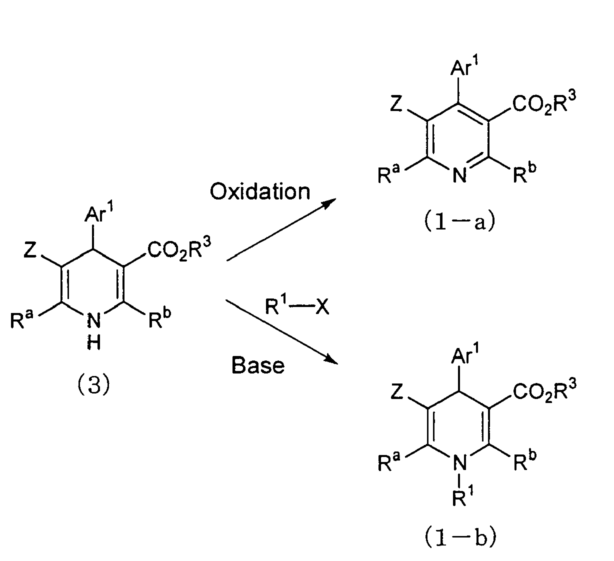

That is, the compound of formula (1-a) in which nitrogen-containing hetero ring moiety is a pyridine ring among the compounds of formula (1) according to the present invention can be produced by oxidizing 1,4-dihydropyridine compound (3) with an oxidizing agent such as nitric acid, nitrous acid, chromic acid, iodine or the like.

In addition, the compound of formula (1-b) in which nitrogen-containing hetero ring moiety is a 1,4-dihydropyridine ring among the compounds of formula (1) according to the present invention can be produced by treating in the presence of a base such as sodium hydroxide or the like 1,4-dihydropyridine compound (3) with R¹⁻X wherein R¹ is as defined above, and X is a leaving group such as chlorine atom, bromine atom, iodine atom, methanesulfonyloxy group, paratoluenesulfonyloxy group, trifluoromethanesulfonyloxy group or the like.

In addition, 1,4-dihydropyridine compounds (3) in which Z is a group of formula (2) can be produced according to the methods described in JP 59-161392 A (1984), JP 60-69089 A (1985), JP 60-248693 A (1985), JP 60-258194 A (1985), JP 61-30591 A (1986), JP 61-37793 A (1986), JP 61-63688 A (1986), JP 61-210092 A (1986), JP 61-254596 A (1986), JP 62-169795 A (1987), JP 62-169796 A (1987), JP 62-195392 A (1987), JP 63-68591 A (1988), JP 63-233992 (1988), JP 1-113398 A (1989) and JP 1-275591 A (1989).

Further, 1,4-dihydropyridine compounds (3) in which Z is CO₂R² can be produced according to the methods described in JP 58-167569 A (1983), JP 55-301 A (1980), JP 57-171968 A (1982), JP 60-97956 A (1985), JP 49-108082 A (1974), JP 52-5777 A (1977), JP 47-34368 A (1972), JP 47-34369 A (1972), JP 52-59161 A (1977), JP 55-9083 A (1980), JP 60-233058 A (1985) and JP 58-67668 A (1983).

Hereinafter, the present invention is described based on examples to which the present invention is not limited at all.
The structural formulae of the compounds used in the examples are as follows:

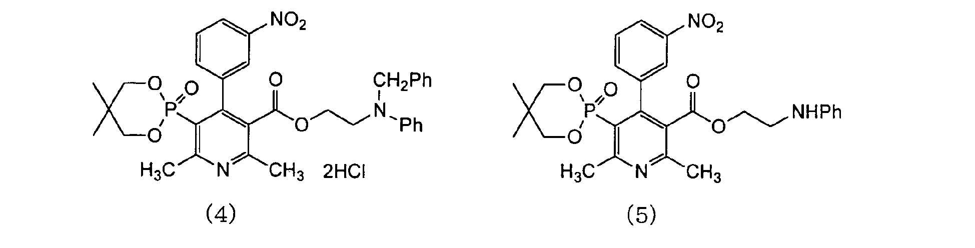

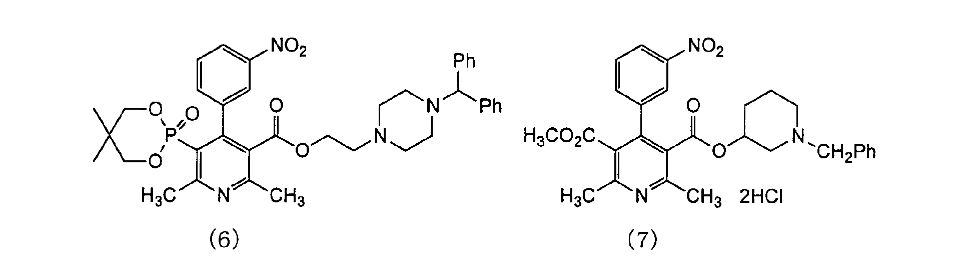

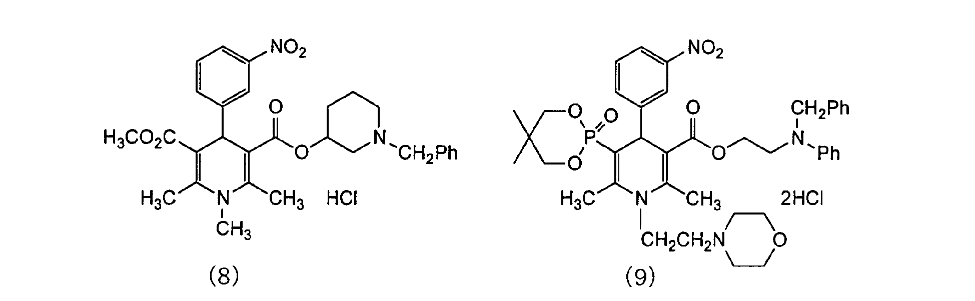

Compound (5) described in the examples was produced according to Oxidation Example 2 indicated in JP 2-138221 (1990), and Compound (6) was produced according to Oxidation Example 1 indicated in JP 2-138221 (1990). Compound (4) described in the examples was produced by benzylating Compound (5) with a benzyl halide and then converting to a hydrochloride.

Compound (7) described in the examples was produced by oxidizing 1,4-dihydropyridine compound (3a) produced by making reference to a production process indicated in JP 57-171968 (1982), and then converting to a hydrochloride as follows. 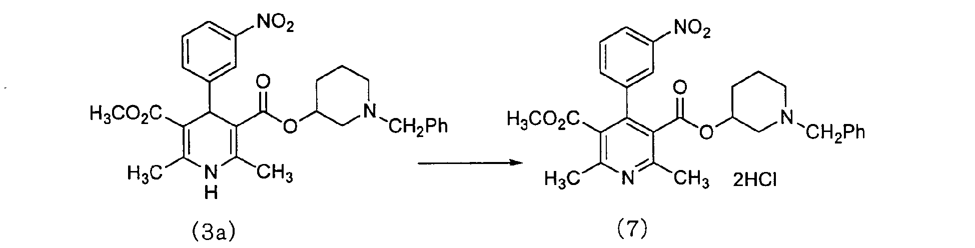 1.0097 g of Compound (3a) was added in 32.5% nitric acid, and the resulting mixture was stirred at 50°C for 15 minutes. After cooling on standing, 300 mL of chloroform and 300 mL of saturated sodium hydrogen carbonate aqueous solution were added in the reaction solution, and then separated into phases. The organic phase was concentrated and then the intended product was separated through silica gel chromatography (eluent, ethyl acetate:hexane=1:1, v/v), and 0.7193 g (76.7%) of pale yellow oily compound was obtained.
NMR spectra (300 MHz, CDCl₃, δ): 1.00-1.20 (1 H, m), 1.40-1.80 (3H, m), 1.80-2.20 (2H, m), 2.30-2.55 (2H, m), 2.61 (3H, s), 2.60 (3H, s), 3.35-3.50 (2H, m), 3.57 (3H, s), 4.75-4.87 (1 H, m), 7.20-7.60 (7H, m), 8.08-8.18 (2H, m).

0.7070 g of the obtained compound was added in 200 mL of chloroform and 100 mL of 1 mol/L hydrochloric acid and separated into phases. The organic phase was dried over anhydrous sodium sulfate, and then concentrated again to obtain 0.7270 g (89.8%) of Compound (7) in a state of colorless solid.

Compound (8) described in the examples was produced by introducing methyl group at 1-position of a hydrochloride of 1,4-dihydropyridine compound (3a) produced by making reference to a production process indicated in JP 57-171968 (1982), and then converting to a hydrochloride. 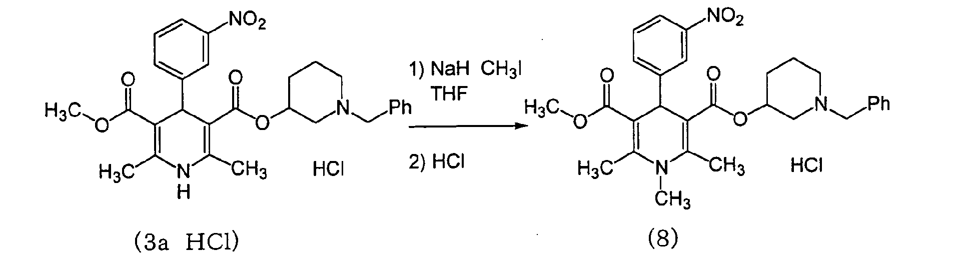 1.01 g (1.86 mmol) of hydrochloride of Compound (3a) was added in 10 mL of tetrahydrofuran containing 529 mg (3.73 mmol) of methyl iodide, and cooled to 3°C. Then, 244 mg (5.59 mmol) of sodium hydride (55%) was added and stirred at 3-6°C for 4 hours. In the resulting reaction mixture, 200 mL of chloroform and 50 mL of water were added, shaken, allowed to stand and separated into phases. The chloroform phase was taken up, the residue obtained by distilling off the solvent was purified through silica gel chromatography (silica gel 40 g, ethyl acetate/hexane=2/3) to obtain 237 mg (24.4%) of the intended N-methyl form as a pale yellow oily product. In 207 mg of the oily product, 200 mL of chloroform and 100 mL of 1 mol/L hydrochloric acid were added, shaken, allowed to stand and separated into phases. The chloroform phase was taken up, dried over 5 g of anhydrous sodium sulfate, and then filtrated and the solvent was distilled off to obtain 246 mg of Compound (8) as a pale yellow solid.
The compound was diastereomer mixture, and the HPLC analysis described below showed mainly two divided peaks of a retention time of 37.88 min. and 39.82 min.
[HPLC Condition]
Column: L-column ODS
Eluent: CH₃CN-0.01 M aq. AcONH₄=3/2(v/v)
Flow rate: 1.0 mL/min.
Temperature: 40°C
Wavelength: 254 nm

Compound (9) described in the examples was produced by introducing a substituent at 1-position of Compound (3b) indicated in Example 25 of JP 63-233992 (1988), and then converting to a hydrochloride (dihydrochloride) as follows. 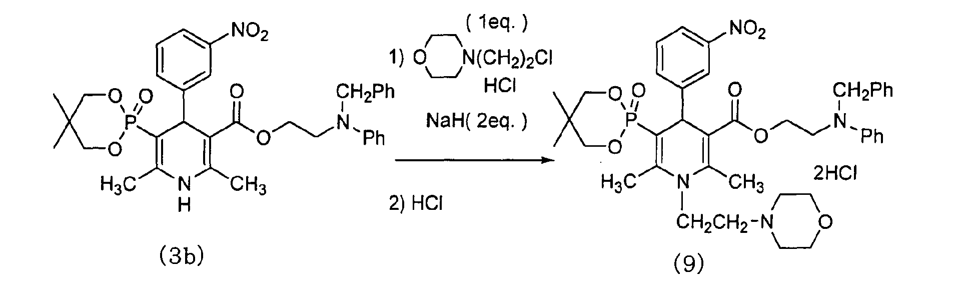 Pharmacological Test Example 1: Effect on T-type Ca channel expressed in mammalian cells (BHK cells)

An electrophysiological evaluation was carried out by use of BHK (baby hamster kidney) cells in which T-type Ca channel (α_{1G}) was expressed according to the method of Wakamori M et al. (Wakamori M et al.: J Biol Chem 273, 34857-34867, 1998) based on the whole cell patch clamp method. Each Ca-ion current was measured through a patch clamp amplifier as an inward current when depolarization pulse (-20 mV) was applied to cells maintained at a membrane potential of -80 mV. Each compound was dissolved in extracellular solution and applied with perfusion. At 5 minutes after the application, any variation in Ca-ion current was measured. The results are shown in Table 1 as Ca current inhibition (%, average value) of the compound of the present invention to Ca current (100%) in vehicle control.

**Table 1**

| Compound | Concentration (µM) | Inhibition (%) | Number of experiments |
|---|---|---|---|
| (4) | 10 | 23.9 | 2 |
| (5) | 10 | 53.9 | 2 |
| (6) | 10 | 43.6 | 2 |
| (7) | 10 | 45.8 | 3 |
| (8) | 10 | 30.0 | 2 |

Pharmacological Test Example 2: Effect on L-type and T-type Ca channel expressed in mammalian cells (BHK cells)

An electrophysiological evaluation was carried out by use of BHK (baby hamster kidney) cells in which L-type Ca channel or T-type Ca channel (α_{1G}) was expressed according to the method of Wakamori M et al. (Wakamori M et al.: J Biol Chem 273, 34857-34867, 1998) based on the whole cell patch clamp method. Each Ca-ion current was measured through a patch clamp amplifier as an inward current when depolarization pulse (10 mV in L-type Ca channel, -20 mV in T-type Ca channel) was applied to cells maintained at a membrane potential of -80 mV. Compound (9) was dissolved in extracellular solution and applied with perfusion. At 5 minutes after the application, any variation in Ca-ion current was measured. The results are shown in Table 2 as Ca current inhibition (%, average value) of the compound of the present invention to Ca current (100%) in vehicle control.

**Table 2**

| Ca channel type | Concentration (µM) | Inhibition (%) | Number of experiments |
|---|---|---|---|
| T-type | 10 | 48.9 | 5 |
| L-type | 10 | 9.9 | 2 |

### Preparation Example 1

Granules containing the following components were prepared.

| | |
|---|---|
| Components Compound of formula (1) | 10 mg |
| Lactose | 700 mg |
| Cornstarch | 274 mg |
| HPC-L | 16 mg |
| | 1000 mg |

The compound of formula (1) and lactose were passed through 60-mesh sieve. Cornstarch was passed through 120-mesh sieve. These components were mixed in a twin-cylinder mixer. Aqueous solution of hydroxypropylcellulose having a low viscosity (HPC-L) was added to the mixed powders, the resulting mixture was kneaded, granulated (extrusion granulation, bore 0.5 to 1 mm), and then dried. The obtained dried granules were passed through a vibrating screen (12/60 mesh) to obtain an intended granules.

### Preparation Example 2

Powders for filling into capsules containing the following components were prepared.

| | |
|---|---|
| Components Compound of formula (1) | 10 mg |
| Lactose | 79 mg |
| Cornstarch | 10 mg |
| Magnesium stearate | 1 mg |
| | 100 mg |

The compound of formula (1) and lactose were passed through 60-mesh sieve. Cornstarch was passed through 120-mesh sieve. These components were mixed with magnesium stearate in a twin-cylinder mixer. 100 mg of 10 times powders were filled into No. 5 hard gelatin capsule.

### Preparation Example 3

Granules for filling into capsules containing the following components were prepared.

| | |
|---|---|
| Components Compound of formula (1) | 15 mg |
| Lactose | 90 mg |
| Cornstarch | 42 mg |
| HPC-L | 3 mg |
| | 150 mg |

The compound of formula (1) and lactose were passed through 60-mesh sieve. Cornstarch was passed through 120-mesh sieve. These components were mixed in a twin-cylinder mixer. Aqueous solution of hydroxypropylcellulose having a low viscosity (HPC-L) was added to the mixed powders, the resulting mixture was kneaded, granulated, and then dried. The obtained dried granules were passed through a vibrating screen (12/60 mesh) to obtain an intended granules. 150 mg of the granules were filled into No. 4 hard gelatin capsule.

### Preparation Example 4

Tablets containing the following components were prepared.

| | |
|---|---|
| Components Compound of formula (1) | 10 mg |
| Lactose | 90 mg |
| Fine crystalline cellulose | 30 mg |
| Magnesium stearate | 5 mg |
| CMC-Na | 15 mg |
| | 150 mg |

The compound of formula (1), lactose, fine crystalline cellulose and CMC-Na (carboxymethylcellulose sodium salt) were passed through 60-mesh sieve and mixed one another. Magnesium stearate was added to the mixed powders to obtain mixed powders for preparation. The powders were subjected to direct compression to obtain 150 mg of tablets.

### Preparation Example 5

Intravenous preparations were prepared as follows.

| | |
|---|---|
| Compound of formula (1) | 100 mg |
| Saturated fatty acid glyceride | 1000 ml |

The compound of formula (1) was dissolved in saturated fatty acid glyceride to obtain an intravenous preparation. Generally, the solution containing the above-mentioned components was intravenously administered to a patient in a rate of 1 ml per minute.

### Industrial Applicability

As the compounds of the present invention have selective T-type calcium channel blocking effect, it is assumed that these compounds can be used for therapy of hypercardia, heart failure, cardiomyopathy, tachycardia-arrhythmia represented by atrial fibrillation, arterial sclerosis, renal disorder represented by nephritis/nephropathy, renal insufficiency, edema, inflammation, hyper-aldosteronism, neurogenic pain, epilepsy, cancer or the like, without adverse effect on blood pressure, cardiac function and Quality of Life. Therefore, the present invention can provide therapeutic agents for the above-mentioned diseases with effectiveness, safety and Quality of Life.

## Claims

1. A T-type calcium channel blocker that is a compound of formula (1), a pharmaceutically acceptable salt thereof or a solvate thereof: 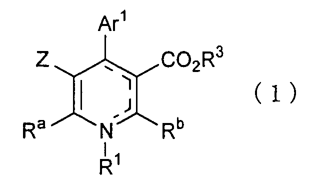 wherein
Ar¹ is phenyl group, pyridyl group, furyl group or 2,1,3-benzoxadiazol-4-yl group (the phenyl group, pyridyl group, furyl group and 2,1,3-benzoxadiazol-4-yl group may be arbitrarily substituted with one or two substituents selected from NO₂ CF₃, Br, Cl, F, C₁₋₂₀alkyl group, OH, OR⁶, OCHF₂, COOR⁶, NH₂, NHR⁶, NR⁶R⁷, CONH₂, CONHR⁶, CONR⁶R⁷, COSR⁶, SR⁶, S(O)R⁶, S(O)₂R⁶, SO₃H, SO₃R⁶, SO₂NH₂, SO₂NHR⁶, SO₂NR⁶R⁷, CN and phenyloxy group, wherein R⁶ and R⁷ are independently of each other C₁₋₆alkyl group;
nitrogen-containing hetero ring moiety is 1,4-dihydropyridine ring or pyridine ring; Z is a group of formula (2) 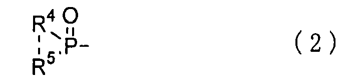 wherein R⁴ and R⁵ are independently of each other OH, C₁₋₆alkoxy group, C₃₋₆alkenyloxy group, C₃₋₆alkynyloxy group, OAr², OANR⁶R⁷, OAN(CH₂Ar²)R⁶, OAOR⁶, OACN, NH₂, NHR⁶, NR⁶R⁷, 1-pyperidinyl group or 1-pyrrolidinyl group, or
R⁴ and R⁵ together are OYO, NHYO, R⁶NYO, NHYNH, R⁶NYNH or R⁶NYNR⁷ wherein R⁶ and R⁷ are as defined above,
Ar² is phenyl group (the phenyl group may be arbitrarily substituted with halogen atom, C₁₋₃alkyl group or C₁₋₃alkoxy group),
A is C₂₋₆alkylene group (the C₂₋₆alkylene group may be arbitrarily substituted with C₁₋₃alkyl group or Ar²), and
Y is straight-chain C₂₋₄alkylene group (the C₂₋₄alkylene group may be arbitrarily substituted with C₁₋₆alkyl group, C₁₋₆alkoxy group, C₁₋₆alkoxycarbonyl group or Ar²), or
Z is CO₂R², wherein R² is C₁₋₆alkyl group (the C₁₋₆alkyl group may be arbitrarily substituted with C₁₋₃alkoxy group);
R^{a} and R^{b} are independently of each other C₁₋₆alkyl group, ANR⁸R⁹, CH₂OANR⁸R⁹, Ar², CH=CHAr², CH₂CH(OH)Ar², CHO, CN, CH₂OH, CH₂OR⁸, AN(CH₂CH₂)₂NR⁸ or NR⁸R⁹, wherein R⁸ and R⁹ are independently of each other hydrogen atom, C₁₋₆alkyl group (the C₁₋₆alkyl group may be arbitrarily substituted with phenyl group, wherein the phenyl group may be arbitrarily substituted with C₁₋₆alkoxy group or halogen atom) or phenyl group (the phenyl group may be arbitrarily substituted with C₁₋₆alkoxy group or halogen atom),
Ar² and A are as defined above;
in case where the nitrogen-containing hetero ring moiety is 1,4-dihydropyridine ring, R¹ is C₁₋₆alkyl group, ANR⁸R⁹, AN(CH₂CH₂)₂NR⁸, AN(CH₂CH₂)₂O, AOR⁸ or benzyl group, wherein R⁸, R⁹ and A are as defined above; and
R³ is hydrogen atom, C₁₋₂₀alkyl group, C₂₋₆alkenyl group or C₂₋₆alkynyl group (C₁₋₂₀alkyl group, C₂₋₆alkenyl group and C₂₋₆alkynyl group may be arbitrarily substituted with phenyl group, wherein the phenyl group may be arbitrarily substituted with C₁₋₆alkoxy group or halogen atom), ANR⁸R⁹ or a group of formula 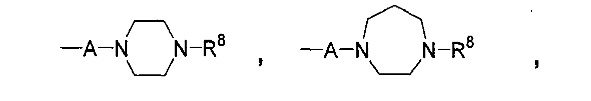 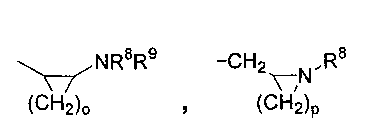 or 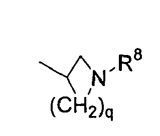 wherein R⁸, R⁹ and A are as defined above,
o and p are independently of each other 3 or 4, and
q is 1,2 or 3.

2. The T-type calcium channel blocker according to claim 1, wherein R³ is ANR⁸R⁹ or a group of formula 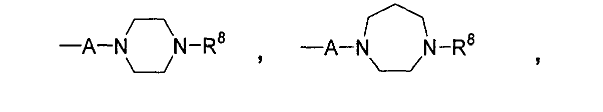 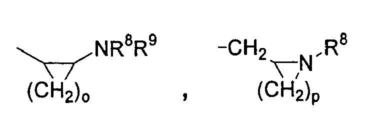 or 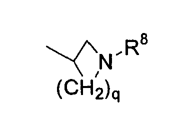 wherein R⁸, R⁹, A, o, q and p are as defined above; and
R⁵ is C₁₋₆alkyl group.

3. The T-type calcium channel blocker according to claim 2, wherein R^{b} is C₁₋₆alkyl group, CN or NH₂.

4. The T-type calcium channel blocker according to claim 1, wherein R^{b} is ANR⁸R⁹, CH₂OANR⁸R⁹ or CH₂CH₂N(CH₂CH₂)₂NR⁸, wherein
A, R⁸ and R⁹ are as defined above;
R³ is C₁₋₂₀alkyl group, C₂₋₆alkenyl group or C₂₋₆alkynyl group (C₁₋₂₀alkyl group,
C₂₋₆alkenyl group and C₂₋₆alkynyl group may be arbitrarily substituted with phenyl group, wherein the phenyl group may be arbitrarily substituted with C₁₋₆alkoxy group or halogen atom); and
R⁵ is C₁₋₆alkyl group.

5. The T-type calcium channel blocker according to any one of claims 1 to 4, wherein the nitrogen-containing hetero ring moiety is 1,4-dihydropyridine ring; and
Z is a group of formula (2).

6. The T-type calcium channel blocker according to claim 5, wherein R⁴ and R⁵ together are OYO, NHYO, R⁶NYO, NHYNH, R⁶NYNH or R⁶NYNR⁷, wherein
Y is straight-chain C₂₋₄alkylene group (the C₂₋₄alkylene group may be substituted with C₁₋₆alkyl group, C₁₋₆alkoxy group, C₁₋₆alkoxycarbonyl group or Ar²).

7. The T-type calcium channel blocker according to claim 6, wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group.

8. The T-type calcium channel blocker according to any one of claims 1 to 4, wherein the nitrogen-containing hetero ring moiety is pyridine ring; and
Z is a group of formula (2).

9. The T-type calcium channel blocker according to claim 8, wherein R⁴ and R⁵ together are OYO, NHYO, R⁶NYO, NHYNH, R⁶NYNH or R⁶NYNR⁷, wherein
Y is straight-chain C₂₋₄alkylene group (the C₂₋₄alkylene group may be arbitrarily substituted with C₁₋₆alkyl group, C₁₋₆alkoxy group, C₁₋₆alkoxycarbonyl group or Ar²).

10. The T-type calcium channel blocker according to claim 9, wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group.

11. The T-type calcium channel blocker according to any one of claims 1 to 4, wherein the nitrogen-containing hetero ring moiety is 1,4-dihydropyridine ring; and
Z is CO₂R².

12. The T-type calcium channel blocker according to claim 11, wherein Ar¹ is phenyl group, 3-nitrophenyl group, 2-nitrophenyl group, 3-chlotophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group.

13. The T-type calcium channel blocker according to any one of claims 1 to 4, wherein the nitrogen-containing hetero ring moiety is pyridine ring; and
Z is CO₂R².

14. The T-type calcium channel blocker according to claim 13, wherein Ar¹ is phenyl group, 3-nitrophenyl group, 2-nitrophenyl group, 3-chlorophenyl group, 2-chlorophenyl group, 3-methoxyphenyl group, 2-methoxyphenyl group, 2-trifluoromethylphenyl group, 3-trifluoromethylphenyl group, 4-pyridyl group, 3-pyridyl group, 2-pyridyl group or 2,3-dichlorophenyl group.

15. A pharmaceutical containing the T-type calcium channel blocker according to claim 1.

16. The pharmaceutical according to claim 15, wherein the pharmaceutical is a therapeutic or preventive agent against a disease for which T-type calcium channel blocking action is effective.

17. The pharmaceutical according to claim 16, wherein the disease is hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer.

18. A method for preventing or treating hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer, comprising administering an effective amount of the compound of formula (1), a pharmaceutically acceptable salt thereof or a solvate thereof according to claim 1.

19. Use of the compound of formula (1), a pharmaceutically acceptable salt thereof or a solvate thereof according to claim 1 for the manufacture of a preventive agent or a therapeutic agent for hypercardia, heart failure, cardiomyopathy, atrial fibrillation, tachyarrhythmia, arterial sclerosis, nephritis, nephropathy, renal disorder, renal insufficiency, inflammation, edema, hyper-aldosteronism, neurogenic pain, epilepsy or cancer.
